(19) Europäisches Patentamt
European Patent Office
Office européen des brevets

(11) **EP 3 975 189 A1**

(12) **EUROPEAN PATENT APPLICATION**
published in accordance with Art. 153(4) EPC

(43) Date of publication:
**30.03.2022 Bulletin 2022/13**

(21) Application number: **20808954.0**

(22) Date of filing: **22.05.2020**

(51) International Patent Classification (IPC):
**G16B 25/10** (2019.01)   **G16B 20/20** (2019.01)
**G16B 30/10** (2019.01)

(52) Cooperative Patent Classification (CPC):
**G16B 20/20; G16B 25/10; G16B 30/10**

(86) International application number:
**PCT/KR2020/006720**

(87) International publication number:
**WO 2020/235972 (26.11.2020 Gazette 2020/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.05.2019 KR 20190059946**

(71) Applicant: **Seoul National University R&DB
Foundation
Seoul 08826 (KR)**

(72) Inventor: **HAN, Buhm
Seoul 01687 (KR)**

(74) Representative: **Cabinet Beau de Loménie
158, rue de l'Université
75340 Paris Cedex 07 (FR)**

(54) **METHOD AND DEVICE FOR PREDICTING GENOTYPE USING NGS DATA**

(57) The present invention relates to a method and device for predicting genotype using NGS data. An embodiment includes: a step for acquiring NGS data on a subject of analysis; a step for applying an NGS-based prediction technique to acquire a first probability; a step for applying an SNP-based prediction technique to acquire a second probability; and a step for predicting the genotype in the NGS data on the subject of analysis on the basis of the first probability and the second probability.

FIG. 1

# Description

Technical Field

The following description relates to a method for predicting a genotype using NGS data.

Background Art

DNA present in the chromosomes in the cell of organisms including humans is a genetic material which is passed on to the offspring during the reproduction and propagation and in the case of humans, DNA inherited from each parent forms pairs of chromosomes. A part of the DNA base sequence which is involved in the phenotypic expression is called genes and the protein is synthesized by the gene expression to form a structure and a function of the organism. A different genotype is determined for every organism due to the difference in DNA base sequence of genes so that single nucleotides which are different for every individual are present in the DNA base sequence of individual belonging to the same species. The genetic diversity caused by the difference in the single nucleotide in the DNA base sequence is called single nucleotide polymorphism (SNP).

A next generation sequencing (NGS) technique is a technique which cuts the DNA or RNA of the organism into small pieces to read the sequences with a machine. In order to find a position in the genome of each sequencing fragment, a mapping task is performed and after finding the positions of all the sequencing fragments, various analyses are performed by analyzing whether the DNA is modified or measuring an amount of DNA which is transcribed into to the RNA. In order to map a genetic material sequencing fragment of a specific organism, a reference genome which is a standard of the corresponding biological genome is necessary and a human reference genome is established by a project such as Human genome project to be continuously updated.

However, in the case of polymorphic genes, that is, a gene (for example, HLA) which may have various genotypes, sequence fragments of a specific organism may have sequences which are very different from the reference so that it is difficult to precisely type a genotype of a gene by mapping to the reference genome from the NGS data. In order to solve the problems in that it is difficult to map the sequence fragments of the gene having a high polymorphism to the reference genome, a method of mapping the sequence fragment to entire sequences of a database in which various known genotypes and sequence information thereof are stored, rather than mapping to only one reference genome. For example, in the case of the human HLA gene, in a public database called IMGT/HLA, various genotypes of HLA which have been known so far and sequence information thereof are stored. However, this method has a problem in that when the depth of the NGS is low, the accuracy is significantly low so that a development of an improved technique which is capable of accurately predicting a genotype even in a low depth of NGS is being demanded.

Disclosure of the Invention

Technical Goals

An aspect provides a technique which accurately analyzes a genotype of a gene having a high polymorphism from NGS data even in a low sequencing depth of NGS data.

The exemplary embodiment discloses a technique of accurately predicting a genotype of an HLA gene which is useful to determine reverse chemotherapy, autoimmune disease risk, organ transplant suitability, and drug side effect.

Technical Solution

According to an aspect, there is provided a method for predicting a genotype using NGS data, the method may include a step for acquiring next generation sequencing (NGS) data on the subject of analysis; a step for mapping the NGS data on the subject of analysis to base sequences having different genotypes for genes on the subject of analysis; a step for acquiring first probabilities that the NGS data on the subject of analysis correspond to genotypes for the genes on the subject of analysis on the basis of the mapping result; extracting SNP data on the subject of analysis from the NGS data; a step for acquiring reference data including a plurality of SNP data having different genotypes for the genes on the subject of analysis; a step for acquiring second probabilities that the SNP data on the subject of analysis corresponds to each of the plurality of genotypes on the basis of the SNP data on the subject of analysis and the reference data; and a step for predicting a genotype of the NGS data on the subject of analysis on the basis of the first probabilities and the second probabilities.

The step for acquiring first probabilities may include: a step for acquiring a length of a mapped base sequence in the NGS data with respect to each of base sequences having different genotypes for the gene on the subject of analysis; and a step for acquiring first probabilities of corresponding to each genotype for the gene on the subject of analysis, on the basis of the length of the mapped base sequence.

The step for predicting a genotype of the NGS data on the subject of analysis may include: a step for acquiring final probabilities that the SNP data on the subject of analysis corresponds each of the plurality of genotypes, by calculating a first probability and a second probability for every genotype; and a step for predicting a genotype corresponding to the highest final probability, among the final probabilities, as a genotype of the NGS data on the subject of analysis.

The step for extracting SNP data on the subject of analysis may further include: a step for detecting SNP from an intergenic region in the NGS data.

The step for acquiring reference data may further include: a step for inserting a marker corresponding into a genotype of the SNP data to each of a plurality of predetermined regions included in SNP data, with respect to each of the plurality of SNP data whose genotype for the gene on the subject of analysis is determined.

The step for acquiring reference data may further include: a step for inserting a binary marker corresponding into a genotype of the SNP data to each of a plurality of exons included in SNP data, with respect to each of the plurality of SNP data whose genotype for the gene on the subject of analysis is determined.

The step for acquiring second probabilities may include: a step for calculating probabilities that the SNP data on the subject of analysis corresponds to the genotypes of the plurality of SNP data, for every region, by inputting the SNP data on the subject of analysis and the reference data to an estimation model; and a step for acquiring second probabilities that the SNP data on the subject of analysis corresponds to each of the plurality of genotypes, on the basis of probabilities for every region.

The step for acquiring second probabilities may include: a step for calculating a genetic distance between a plurality of markers corresponding to the plurality of genotypes; and a step for acquiring second probabilities that the SNP data on the subject of analysis corresponds to each of the plurality of genotypes on the basis of the SNP data on the subject of analysis, the reference data, and the genetic distance.

The step for acquiring second probabilities may include: a step for sampling the SNP data on the subject of analysis and the plurality of SNP data; a step for calculating interstate transition probability corresponding to the plurality of genotypes in the hidden Markov model, on the basis of the sampled data; a step for acquiring an interstate genetic distance by converting the interstate transition probability; and a step for acquiring second probabilities that the SNP data on the subject of analysis corresponds to each of the plurality of genotypes on the basis of the genetic distance, the reference data, and the SNP data on the subject of analysis.

The SNP data on the subject of analysis may include: at least some of DNA base sequences of a user on a subject of analysis; and information of the at least some SNP included in the at least some DNA base sequences.

Each SNP data included in the reference data may include: a DNA base sequence of a corresponding genotype; information of SNP included in the DNA base sequence; and markers inserted into a plurality of predetermined regions in the DNA base sequence.

According to an aspect, a device for predicting a genotype using NGS data includes a memory which stores reference data including a plurality of SNP data in which a genotype of a gene on the subject of analysis is determined; and at least one processor which acquires next generation sequencing (NGS) data on the subject of analysis, maps the NGS data on the subject of analysis to base sequences having different genotypes for genes on the subject of analysis, acquires first probabilities that the NGS data on the subject of analysis correspond to genotypes for the genes on the subject of analysis on the basis of the mapping result, extracts SNP data on the subject of analysis from the NGS data, acquires reference data including a plurality of SNP data into which a marker corresponding to a genotype for the gene on the subject of analysis is inserted, acquires second probabilities that the SNP data on the subject of analysis corresponds to each of the plurality of genotypes on the basis of the SNP data on the subject of analysis and the reference data, and predicts a genotype of the NGS data on the subject of analysis on the basis of the first probabilities and the second probabilities.

When the first probabilities are acquired, the processor acquires a length of the mapped base sequence in the NGS data, with respect to each of base sequences having different genotypes for the gene on the subject of analysis and may acquire the first probabilities of corresponding to each genotype for the gene on the subject of analysis, on the basis of the length of the mapped base sequence.

When the genotype of the NGS data on the subject of analysis is predicted, the processor acquires final probabilities that the SNP data on the subject of analysis corresponds each of the plurality of genotypes, by calculating a first probability and a second probability for every genotype and may predict a genotype corresponding to the highest final probability, among the final probabilities, as a genotype of the NGS data on the subject of analysis.

When the SNP data on the subject of analysis is extracted, the processor may detect SNP from an intergenic region in the NGS data.

When the reference data is acquired, the processor may insert a marker corresponding to a genotype of the SNP data into each of a plurality of predetermined regions included in SNP data, with respect to each of the plurality of SNP data whose genotype for the gene on the subject of analysis is determined.

When the second probabilities are acquired, the processor calculates probabilities that the SNP data on the subject of analysis corresponds to the genotypes of the plurality of SNP data, for every region, by inputting the SNP data on the subject of analysis and the reference data to an estimation model and may acquire second probabilities that the SNP data on the subject of analysis corresponds to each of the plurality of genotypes, on the basis of probabilities for every region.

When the second probabilities are acquired, the processor calculates a genetic distance between a plurality of markers corresponding to the plurality of genotypes and may acquire second probabilities that the SNP data on the subject of analysis corresponds to each of the plurality of genotypes on the basis of the SNP data on the subject of analysis, the reference data, and the genetic distance.

The gene on the subject of analysis is a HLA gene, the plurality of genotypes includes a plurality of genotypes defined in the HLA gene, and the NGS data on the subject of analysis may include a base sequence of the HLA gene.

Brief Description of Drawings

FIG. 1 is a view illustrating a flowchart of a method for predicting a genotype using NGS data according to an exemplary embodiment.

FIG. 2 is a view illustrating a pipeline of a next generation sequencing technique.

FIG. 3 is a view for explaining mapping of NGS.

FIG. 4 is a view for explaining a genotype predicting method according to an NGS based method according to an exemplary embodiment.

FIG. 5 is a view for explaining a method of acquiring a second probability by applying an SNP based prediction technique according to an exemplary embodiment.

FIG. 6 is a view for explaining SNP.

FIG. 7 is a view for explaining a base sequence structure of a specific gene locus in a chromosome.

FIG. 8 is a view for explaining a method for predicting a genotype of SNP data on a subject of analysis, by applying an estimation model according to an exemplary embodiment.

FIG. 9 is a view illustrating an example of a specific operation of an estimation model according to an exemplary embodiment.

Best Mode for Carrying Out the Invention

Hereinafter, exemplary embodiments will be described in detail with reference to the accompanying drawings. However, various changes may be applied to the exemplary embodiments so that the scope of the application is not restricted or limited by the exemplary embodiments. It should be understood that all changes, equivalents, or substitutes of exemplary embodiments are included in the scope of the rights.

Terms used in the exemplary embodiment are used only for illustrative purposes only, but should not be interpreted as an intention to limit the invention. A singular form may include a plural form if there is no clearly opposite meaning in the context. In the present specification, it should be understood that terminology "include" or "have" indicates that a feature, a number, a step, an operation, a component, a part or the combination those of described in the specification is present, but do not exclude a possibility of presence or addition of one or more other features, numbers, steps, operations, components, parts or combinations, in advance.

If it is not contrarily defined, all terms used herein including technological or scientific terms have the same meaning as those generally understood by a person with ordinary skill in the art. Terms defined in generally used dictionary shall be construed that they have meanings matching those in the context of a related art, and shall not be construed in ideal or excessively formal meanings unless they are clearly defined in the present application.

Further, in description with reference to accompanying drawings, the same components are denoted by the same reference numerals regardless of the reference numeral and a duplicated description thereof will be omitted. In description of an exemplary embodiment, if it is determined that detailed description for a related art may unnecessarily blur the gist of the exemplary embodiment, the detailed description will be omitted.

Further, in the description of the components of the exemplary embodiment, a terminology such as a first, a second, A, B, (a), (b) may be used. However, such terminologies are used only to distinguish a component from another component but nature, a sequence or an order of the component is not limited by the terminologies. If it is described that a component is "connected" or "coupled" to another component, it is understood that the component is directly connected or coupled to the other component but another component may be "connected" or "coupled" between the components.

A component including a common function to a component included in any one exemplary embodiment will be described with the same title in another exemplary embodiment. If it is not contrarily defined, description of any one exemplary embodiment may be applied to another exemplary embodiment and a detailed description of overlapping parts will be omitted.

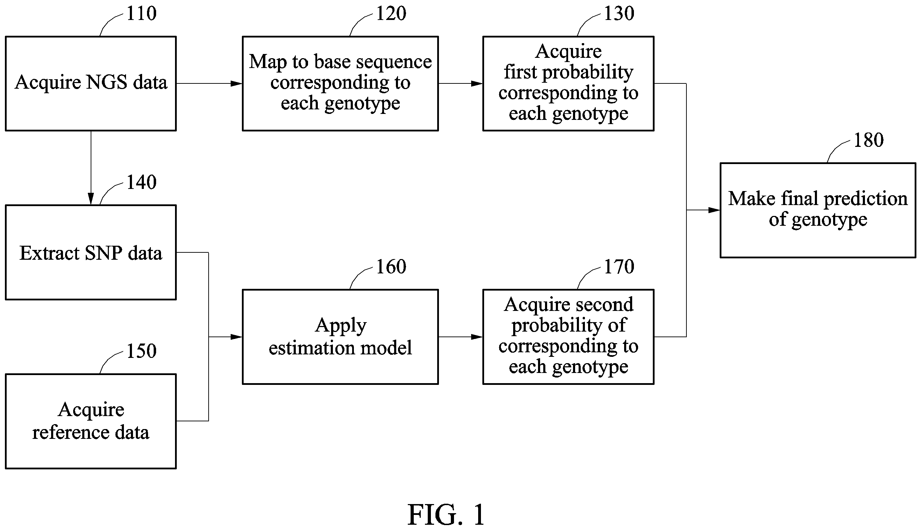

FIG. 1 is a view illustrating a flowchart of a method for predicting a genotype using NGS data according to an exemplary embodiment.

Referring to FIG. 1, a method for predicting a genotype using NGS data according to an exemplary embodiment includes a step 110 for acquiring NGS data on a subject of analysis, a step 130 for applying an NGS based prediction technique to acquire a first probability, a step 170 for applying an SNP based prediction technique to acquire a second probability, and a step 180 for predicting the genotype in the NGS data on the subject of analysis on the basis of the first probability and the second probability. According to an exemplary embodiment, the step for applying an NGS based prediction technique to acquire a first probability may include a step 120 for mapping NGS data to a base sequence corresponding to each genotype and a step 130 for acquiring a first probability of each genotype on the basis of the mapping result. The step for applying an SNP based prediction technique to acquire a second probability according to the exemplary embodiment may include a step 140 for extracting SNP data on a subject of analysis from the NGS data on a subject of analysis, a step 150 for acquiring reference data including a plurality of SNP data into which markers corresponding to a genotype of a gene on a subject of analysis is inserted, and a step 170 for acquiring a second probability for each genotype based on the SNP data on the subject of analysis and the reference data. The step 170 for acquiring a second probability for each genotype according to the exemplary embodiment may include a step 160 for applying an estimation model to the SNP data on the subject of analysis and the reference data.

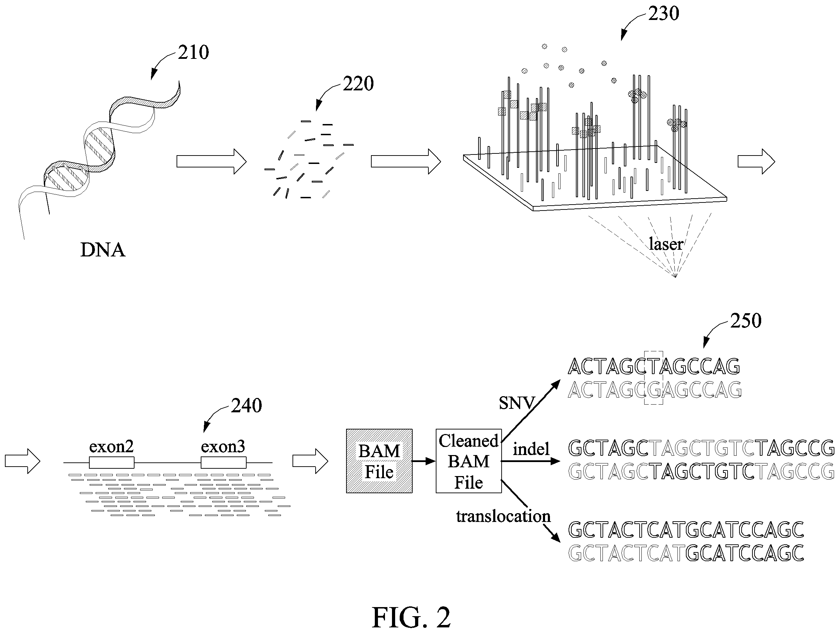

A next generation sequencing (NGS) technique is a technique which cuts the DNA or RNA of the organism into small pieces to read the sequences with a machine. The pipeline of the next generation sequencing technique will be described with reference to FIG. 2. Referring to FIG. 2, the next generation sequencing technique extracts a DNA of an organism (210), cuts the DNA into short sequence fragments (220), to perform sequencing on each sequence fragment to analyze the base included in the sequence (230). The sequencing method includes pyrosequencing and alumina sequencing. After the sequencing, in order to identify a position of each sequence fragment in the genome, mapping to align each sequence fragment on the basis of the reference genome is performed (240) to identify the position of sequence fragments in the genome. After identifying the positions of all sequence fragments, various analyses of analyzing whether the DNA is modified or measuring an amount of DNA which is transcribed into RNA may be performed (250).

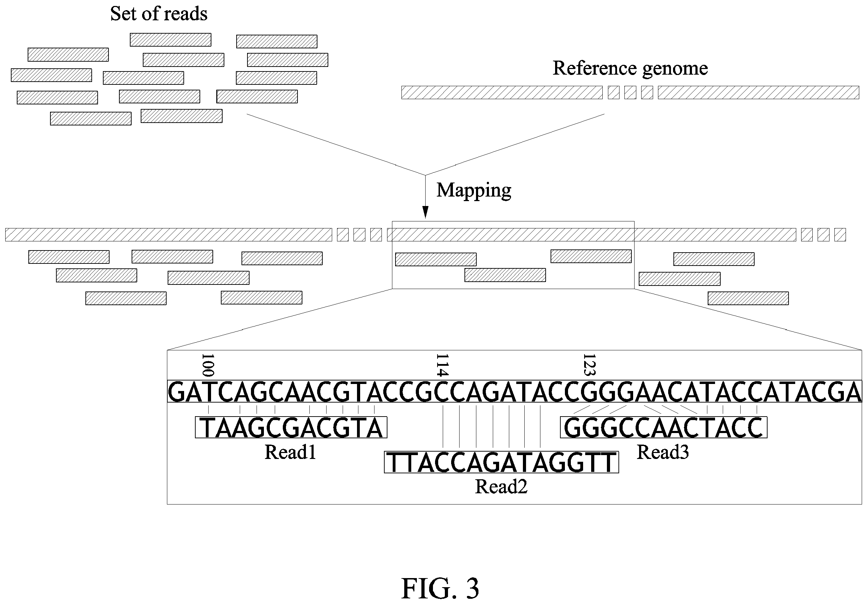

FIG. 3 is a view for explaining mapping of NGS. Referring to FIG. 3, the reference genome is a genome which serves as a standard for a genome of a specific organism for mapping sequence fragments of a genetic material of the corresponding organism. Locations of the sequence fragments (sets of reads) in the genome are found by mapping the sequence fragments (sets of reads) whose base is analyzed by the sequencing to the sequences of the reference genome. At this time, the location in the reference genome corresponding to the sequence matching the set of reads is considered as a location of the set of reads. That is, the mapping may correspond to a process of aligning the set of reads in the location in the genome. As a sequencing result, the number of repetition of a specific base, that is, a degree to which a specific base is repeated is called a sequencing degree or a sequencing depth and when the number of repetition is n, the sequencing depth is expressed by nx. The accuracy of the mapping result may vary depending on the sequencing depth so that when the sequencing depth is low (for example, $10\times$ or lower), there may be parts of reference genomes which are not mapped, and the number of mapped set of reads in a mapped region may be small so that the mapping accuracy is lowered.

Referring to FIG. 1 again, the NGS data according to the exemplary embodiment is base sequence data of an organism acquired by applying next generation sequencing (NGS), and for example, entire base sequence data of organism according to whole genome sequencing technique, base sequence data of a protein coding region in the gene according to exome sequencing, and base sequence data of a targeted region according to targeted sequencing may correspond thereto. The NGS data on the subject of analysis according to the exemplary embodiment may include data acquired by performing the sequencing of cutting base sequence data for a gene on a subject of analysis extracted from the organism into short sequence fragments to analyze the base included in each sequence fragment.

Hereinafter, the base sequence will be described as a DNA base sequence, but the base sequence data according to the exemplary embodiment may include not only the DNA base sequence data, but also RNA base sequence data.

In the DNA base sequence, bases which are components of nucleotides which are basic units of DNA are sequentially arranged. The base which configures the nucleotides corresponds to any one of adenine (A), thymine (T), guanine (G), and cytosine (C). The DNA exists as chromosomes in cells and humans have 23 pairs of chromosomes, one inherited from each parent. Chromosomes which configure one chromosome pair are called homologous chromosomes and one homologous chromosome which configures one chromosome pair is configured by a paternal DNA base sequence and the other homologous chromosome is configured by a maternal DNA base sequence. The gene may include some DNA base sequences which is involved in the phenotypic expression in the chromosomes.

The gene of the organism is determined by some of DNA base sequences of the organism and an expressed phenotype may vary depending on the difference in the base sequence of a corresponding gene locus in the chromosome of the organism. A base sequence of the corresponding gene locus which determines a genotype of a specific gene to be different is called an allele. A genotype may be defined by a phenotype which is expressed by a base sequence of a specific gene locus of the organism. That is, the genotype refers to a genetic character which is expressed by the difference of the base sequence in the living organism and corresponds to a type of allele. There may be differences in genotypes between homogeneous individuals in the same gene and the occurrence of various genotypes is referred to as polymorphism. For example, a human HLA gene includes a plurality of gene loci, and there are dozens of alleles of one gene locus so that it is classified as a gene with a high polymorphism.

A gene located in the same position of the homologous chromosomes which form one chromosome pair determines one phenotype and genes in the homologous chromosomes may have different genotypes. For example, a HLA gene of a person is located on chromosome 6, an X gene of one homologous chromosome of the pair of chromosome 6 corresponds to a genotype A and an X gene of the other homologous chromosome corresponds to a B type. Accordingly, the DNA base sequences extracted from the gene of one individual correspond to DNA base sequence pairs each having a genetic character and is expressed by one pair of genotype.

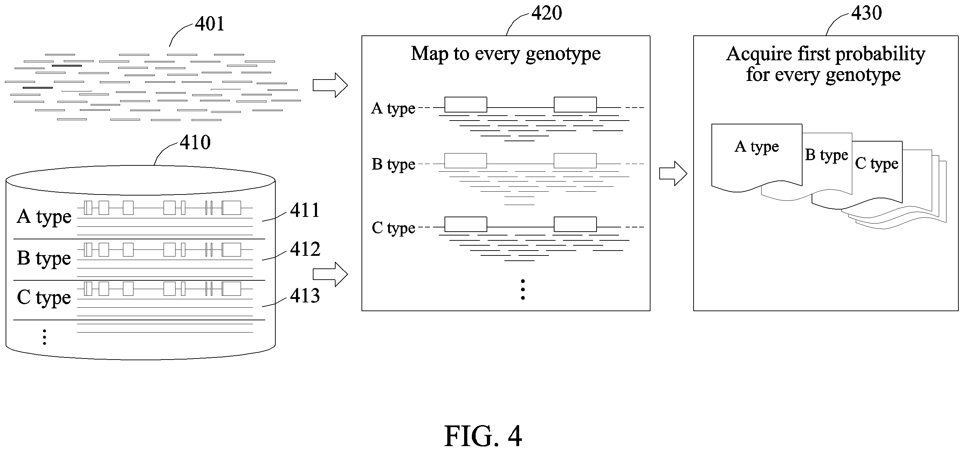

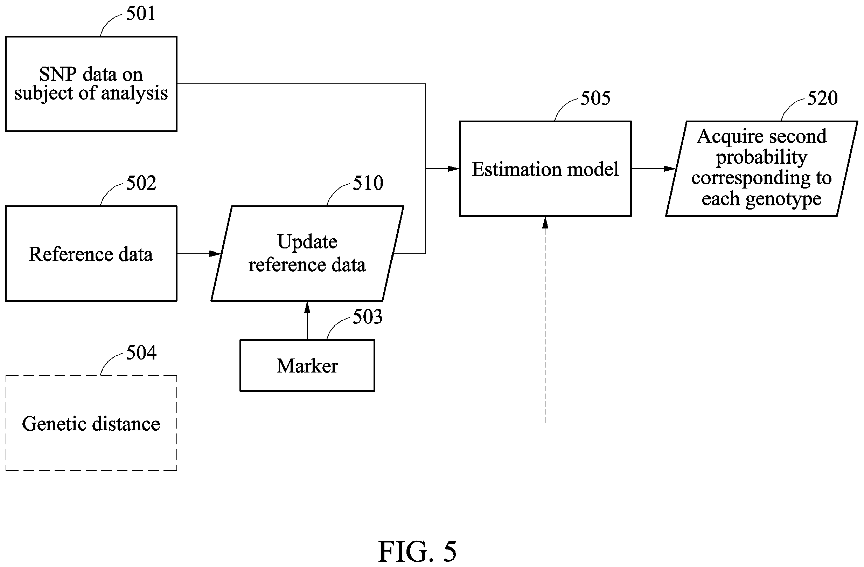

A first probability according to an NGS based prediction technique and a second probability according to an SNP based prediction technique may be acquired based on the NGS data acquired in the step 110 according to the exemplary embodiment. The method for acquiring the first probability according to the NGS based prediction technique according to the exemplary embodiment will be described in detail with reference to FIG. 4 and the method for acquiring the second probability according to the SNP based prediction technique according to the exemplary embodiment will be described in detail with reference to FIG. 5.

The step 180 for making final prediction of a genotype according to the exemplary embodiment corresponds to a step for predicting a genotype of NGS data on a subject of analysis based on the first probabilities and the second probabilities of corresponding to a plurality of genotypes. According to an exemplary embodiment, the step 180 for making final prediction of a genotype may include a step for acquiring final probabilities that SNP data on the subject of analysis corresponds to each of a plurality of genotypes by calculating the first probability and the second probability for every genotype. The calculating method of the first probability and the second probability according to the exemplary embodiment may include various calculating methods according to a predetermined reference. For example, when A(01:01) is considered as a final probability that there is 01:01 genotype in the HLA-A gene, if A(01:01)[1] and A(01:01)[2] are a first probability and a second probability, respectively, A(01:01) may be defined as a weighted average probability a*A(01:01)[1] + (1-a)*A(01:01)[2]. Here, a weight a may be determined by a value of 0 or larger and 1 or smaller based on the importance of information according to the NGS based prediction and the importance of information according to the SNP based prediction and an optimal weight may be acquired based on training data. Here, the training data may include NGS data having a deep sequencing depth for which the genotype is already known. As an example of the method for acquiring an optimal weight based on the training data, data having a less deeper depth may be made by arbitrarily sampling from NGS data having a deep sequencing depth so that if data to be predicted has a depth U, virtual data having a depth U may be made from the NGS data included in the training data. A genotype is predicted on this virtual data with a weight between 0 and 1, according to the exemplary embodiment so that it is possible to distinguish which weight is an optimal weight having a highest accuracy for predicting a genotype. The step 180 according to the exemplary embodiment may include a step for predicting a genotype corresponding to the highest final probability among final probabilities acquired for every genotype as a genotype of NGS data on the subject of analysis.

FIG. 4 is a view for explaining a genotype predicting method according to an NGS based method according to an exemplary embodiment.

Referring to FIG. 4, the method for applying an NGS based prediction technique according to the exemplary embodiment to acquire a first probability may include a step 420 for mapping NGS data to a base sequence corresponding to each genotype and a step 430 for acquiring a first probability of each genotype based on the mapping result.

The step 420 for mapping NGS data 401 to base sequences 411, 412, and 413 corresponding to each genotype according to the exemplary embodiment may correspond to a step for mapping NGS data on the subject of analysis to base sequences having different genotypes for a gene on the subject of analysis. In other words, the step 420 according to the exemplary embodiment may correspond to a step for mapping the NGS data on the subject of analysis to each of the plurality of base sequences with the plurality of base sequences 411, 412, and 413 whose genotype for the gene on the subject of analysis is determined as a reference genome. Here, the plurality of base sequences 411, 412, and 413 which are reference genomes for mapping may include base sequences in which genotypes for the gene on the subject of analysis are differently determined. For example, when genotypes for the gene on the subject of analysis include A type, B type, and C type, the NGS data on the subject of analysis may be mapped to a base sequence 411 corresponding to the A type, a base sequence 412 corresponding to the B type, and a base sequence 413 corresponding to the C type, respectively. According to the exemplary embodiment, a database 410 of base sequences corresponding to genotypes for genes on the subject of analysis may be used. For example, when the gene on the subject of analysis is a human HLA gene, based on the IMGT/HLA database in which base sequences corresponding to various genotypes are stored, the mapping step may be performed with the base sequence corresponding to each genotype stored in the IMGT/HLA as a reference genome.

The step 430 for acquiring a first probability for every genotype according to the exemplary embodiment may correspond to a step for acquiring first probabilities that the NGS data on the subject of analysis corresponds to genotypes for the gene on the subject of analysis. In other words, the first probability that the NGS data on the subject of analysis corresponds to a specific genotype is a probability acquired based on a result of mapping the NGS data on the subject of analysis with the base sequence corresponding to the genotype as the reference genome, and the step 430 for acquiring a first probability for every genotype may correspond to the step for acquiring a first probability for each of the plurality of genotypes for the gene on the subject of analysis. For example, when the genotype for the gene on the subject of analysis includes an A type, a B type, and a C type, a first probability that the NGS data on the subject of analysis corresponds to the A type, a first probability that the NGS data on the subject of analysis corresponds to the B type, and a first probability that the NGS data on the subject of analysis corresponds to the C type may be acquired, based on the mapping result for every genotype.

According to the exemplary embodiment, the first probability of corresponding to a specific genotype may be acquired by acquiring a length of the base sequence mapped to the base sequence corresponding to the corresponding genotype from the NGS data on the subject of analysis to compare an overall length of the base sequence corresponding to the corresponding genotype and a length of the mapped base sequence. Here, the length of the mapped base sequence may refer to the number of bases mapped to the reference genome. Further, the length of the mapped base sequence may include the number of sequence fragments in the NGS data on the subject of analysis mapped to the reference genome.

According to the exemplary embodiment, the NGS data on the subject of analysis may be processed to analyze the genotype for a maternal DNA base sequence and a paternal DNA base sequence.

FIG. 5 is a view for explaining a method for applying an SNP based prediction technique to acquire a second probability according to an exemplary embodiment.

Referring to FIG. 5, the method for applying an SNP based prediction technique to acquire a second probability according to the exemplary embodiment includes a step for extracting SNP data 501 on the subject of analysis from NGS data on the subject of analysis, a step 510 for updating reference data by inserting a marker 503 into reference data 502, and a step 520 for acquiring a second probability that the SNP data on the subject of analysis corresponds to each genotype. The step 520 for acquiring a second probability that the SNP data on the subject of analysis corresponds to each genotype may include a step for acquiring a second probability that the SNP data on the subject of analysis corresponds to each genotype by inputting the SNP data on the subject of analysis and the reference data to an estimation model 505.

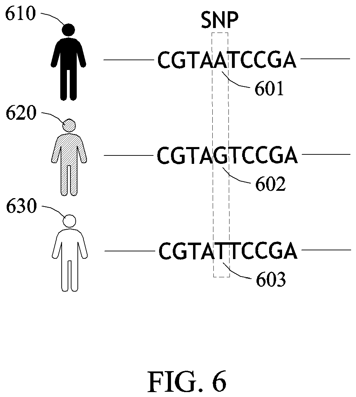

Single nucleotide polymorphism (SNP) refers to a position of a single nucleotide in the DNA base sequence which is different for every living organism. Different organisms belonging to the same species may have different genetic characters due to difference in the base sequence expressed in the SNP. For example, referring to FIG. 6, three DNA base sequences may correspond to some base sequences corresponding to the same position, among the DNA base sequences of individuals 610, 620, and 630 belonging to the same species. In the base sequences of the individuals of FIG. 2, CGTA and TCCGA are common, but fifth bases are A (601), G (602), and T (603) which are different for every individual.

That is, in FIG. 2, the position of the fifth base is referred to as SNP. The character of the individual may vary due to the difference in some single nucleotides among the DNA base sequences.

Referring to FIG. 5 again, the SNP data according to the exemplary embodiment may include at least some DNA base sequences of a specific gene locus of a specific biological individual and information of at least some SNP included in at least some DNA base sequences. Some DNA base sequences included in the SNP data according to the exemplary embodiment may include single bases which are different from the DNA base sequence of another individual belonging to the same species. The SNP information included in the SNP data according to the exemplary embodiment may include position information of the single nucleotides which are different from the DNA base sequence of another individual belonging to the same species, among the DNA base sequences included in the SNP data.

The DNA base sequence included in the SNP data according to the exemplary embodiment may include maternal DNA base sequences and paternal DNA base sequences. Hereinafter, the maternal DNA base sequences and the paternal DNA base sequences are referred to as pairs of the DNA base sequences. As long as the DNA base sequence is not limited to referring to only any one of the paternal DNA base sequence and the maternal DNA base sequence, the DNA base sequences may refer to the maternal DNA base sequence and the paternal DNA base sequence.

The SNP data 501 on the subject of analysis according to the exemplary embodiment may correspond to SNP data extracted by a method for detecting a variant such as SNP from the base sequence data, such as variant calling from the NGS data for a specific gene of a user on a subject of analysis. The SNP data 501 on the subject of analysis according to the exemplary embodiment may include at least one DNA base sequence of a specific gene among DNA base sequences of the user on the subject of analysis and information of at least some SNP included in at least some DNA base sequences. As described above, the DNA base sequence included in the data on the subject of analysis according to the exemplary embodiment may include maternal DNA base sequences and paternal DNA base sequences.

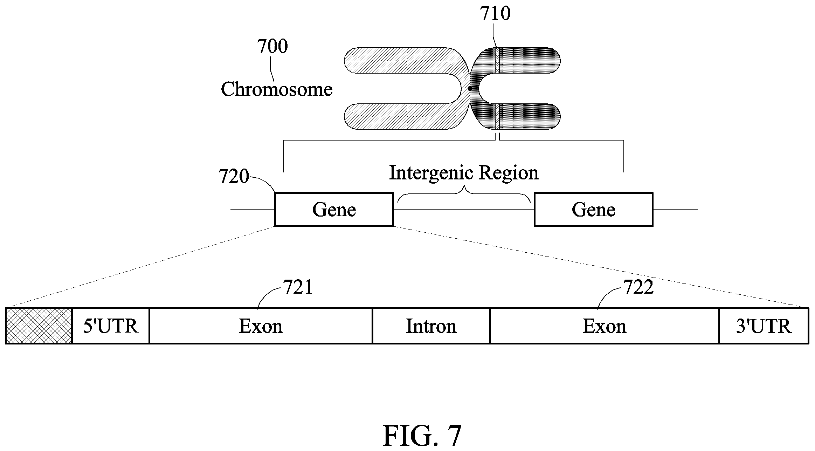

According to the exemplary embodiment, the SNP data on the subject of analysis may include SNP data detected from an intergenic region of the gene on the subject of analysis. The intergenic region refers to base sequences which are not expressed in the DNA base sequences, and referring to FIG. 7, a region other than the gene region in the base sequence of a specific gene locus of the chromosome corresponds to the intergenic region. For example, when the gene on the subject of analysis is a HLA gene, the SNP data 501 on the subject of analysis may correspond to SNP data extracted from the intergenic region in the NGS data regarding the HLA gene by the variation detecting method. In this case, the SNP data 501 on the subject of analysis may include DNA base sequence pairs including single nucleotides which are extracted from the HLA gene located in a specific position of human chromosome 6 and are different for every human and include the position information of the single nucleotides which may show different types of nucleotides for every person.

The step for extracting SNP data on the subject of analysis from the NGS data on the subject of analysis according to the exemplary embodiment may include a step for paging SNP data on the subject of analysis to analyze a genotype for each maternal DNA base sequence and paternal DNA base sequence to separate two haploid data and a step for acquiring two diploid data in which haploid data and replica data of the corresponding haploid data form one pair, by replicating two haploid data. The paging according to the exemplary embodiment may refer to an operation of separating the pairs of the DNA base sequences into the maternal DNA base sequences and the paternal DNA base sequences. The haploid data according to the exemplary embodiment may refer to SNP data including only one DNA base sequence of the paternal DNA base sequence and the maternal DNA base sequence. The diploid data according to the exemplary embodiment may refer to SNP data including the pairs of the same DNA base sequences made by replicating the DNA base sequence included in the haploid data.

For example, when the SNP data on the subject of analysis includes a paternal DNA base sequence a and a maternal DNA base sequence b, the haploid data which are separated into two by paging the SNP data on the subject of analysis may refer to SNP data only including the paternal DNA base sequence a and SNP data only including the maternal DNA base sequence b. In the same example, two diploid data in which the haploid data and replica data of the corresponding diploid form one pair may refer to SNP data including a pair of DNA base sequences configured by two paternal DNA base sequences a and SNP data including a pair of DNA base sequences configured by two maternal DNA base sequences b.

Referring to FIG. 5 again, the reference data 502 according to the exemplary embodiment includes SNP data in which a genotype is determined by any one of a plurality of genotypes for the gene on the subject of analysis. For example, when the SNP data on the subject of analysis is extracted from the HLA gene, the SNP data included in the reference data 502 includes at least some DNA base sequences of the HLA gene and information of at least some SNP included in at least some DNA base sequences, and at least some DNA base sequences may include single nucleotides which are different from the base sequence of another individual. According to the exemplary embodiment, the SNP data included in the reference data 502 may include the SNP data extracted from the intergenic region.

The SNP data according to the exemplary embodiment whose genotype is determined may include at least one SNP data corresponding to any one of a plurality of genotypes defined in the gene from which the SNP data on the subject of analysis is extracted. In other words, the SNP data according to the exemplary embodiment whose genotype is determined may correspond to a pair of genotypes corresponding to any one of the plurality of genotypes defined in the gene on the subject of analysis. The SNP data included in the reference data 502 according to the exemplary embodiment includes a pair of DNA base sequences configured by two DNA base sequences and each DNA base sequence corresponds to any one genotype among the plurality of genotypes defined in the gene on the subject of analysis. That is, the pair of genotypes corresponding to the SNP data included in the reference data 502 may correspond to a pair of genotypes corresponding to the DNA base sequences which configure the DNA base sequence pair included in the SNP data.

For example, when genotypes of A type, B type, and C types are defined in the gene from which the SNP data on the subject of analysis is extracted, the first SNP data included in the reference data 502 may include a pair of a DNA base sequence corresponding to the A type and a DNA base sequence corresponding to the B type and the second SNP data included in the reference data 502 may include a pair of a DNA base sequence corresponding to the A type and a DNA base sequence corresponding to the C type. In this case, the pair of the genotypes corresponding to the first SNP data included in the reference data 502 may correspond to (A type, B type) and the pair of the genotypes corresponding to the second SNP data included in the reference data 502 may correspond to (A type, C type).

The step 510 for updating the reference data 502 according to the exemplary embodiment may correspond to a step for updating reference data 502 by inserting a marker 503 corresponding to the pair of genotypes of the corresponding SNP data into a plurality of predetermined regions included in the corresponding SNP data, so as to correspond to the SNP data included in the reference data 502. The step 510 for updating reference data 502 according to the exemplary embodiment may further include a step for determining markers 503 corresponding to the genotypes of the plurality of SNP data, before inserting the marker 503.

The marker 503 according to the exemplary embodiment may include a marker defined so as to correspond to each of the plurality of predefined genotypes of the gene on the subject of analysis. For example, when the plurality of genotypes predefined in the gene on the subject of analysis corresponds to the A type, the B type, and the C type, the marker 503 according to the exemplary embodiment may include a first marker defined to correspond to the A type, a second marker defined to correspond to the B type, and a third marker defined to correspond to the C type.

The marker 503 according to the exemplary embodiment may include a binary marker indicating whether there is a DNA base sequence corresponding to the genotype corresponding to the marker in the SNP data. If the DNA base sequence included in the SNP data corresponds to a genotype corresponding to the binary marker, the binary marker according to the exemplary embodiment may be represented by 1. Otherwise, the binary marker may be represented by 0. For example, when the first SNP data corresponds to a pair (A type, B type) of the genotype, a first marker defined to correspond to the A type may be represented by (1, 0), a second marker corresponding to the B type may be represented by (0, 1) and a third marker corresponding to the C type may be represented by (0, 0).

According to the exemplary embodiment, the marker 503 may be represented by a tuple of the binary markers (for example, the first binary marker, the second binary marker, and the third binary marker) corresponding to the genotypes of the gene on the subject of analysis, corresponding to one base sequence included in the SNP data. For example, when one base sequence included in the SNP data corresponds to the genotype A, the marker 503 of the corresponding base sequence may be represented by (1, 0, 0) and when the other base sequence corresponds to the genotype B, the marker 503 of the corresponding base sequence may be represented by (0, 1, 0).

In the step 501 for updating reference data 502 according to the exemplary embodiment, the plurality of predetermined regions included in the SNP data may refer to a plurality of regions corresponding to a predetermined position and range in the DNA base sequence included in the SNP data. According to the exemplary embodiment, the plurality of regions may include a plurality of exon regions. The exon refers to a region of the DNA base sequence which is synthesized to protein and there is a plurality of exons in the DNA base sequence of one gene.

For example, referring to FIG. 7, the gene on the subject of analysis may correspond to a DNA base sequence located in a specific position 710 of the chromosome 700. The gene on the subject of analysis may be involved in synthesizing a plurality of proteins and is divided into a plurality of sections according to the protein to be synthesized. A DNA base sequence corresponding to one gene region 720 which synthesizes a specific protein, among the DNA base sequences of the gene on the subject of analysis, may include a plurality of exons 721 and 722 which is involved in synthesizing the specific protein.

Referring to FIG. 5 again, in the step 510 for updating reference data 502 according to the exemplary embodiment, the insertion of the marker 503 corresponding to the pair of the genotypes of the SNP data into the plurality of predetermined regions included in the SNP data refers to the encoding of the plurality of predetermined regions with the marker 503. For example, when the genotypes of the gene on the subject of analysis are defined as the A type, the B type, and the C type, if the first SNP data included in the reference data 502 corresponds to the pair of the genotypes (A type, B type), the DNA base sequences included in the plurality of predetermined regions, among the DNA base sequences included in the first SNP data may be encoded with a binary marker (1, 0) corresponding to the A type, a binary marker (0, 1) corresponding to the B type, and a binary marker (0, 0) corresponding to the C type.

According to the exemplary embodiment, when the plurality of regions of the step 510 corresponds to the plurality of exons, a marker 503 corresponding to the pair of the genotypes of the SNP data may be inserted into the DNA base sequence included in each exon. For example, referring to FIG. 7, when the SNP data included in the reference data includes the DNA base sequence of FIG. 3, the DNA base sequences included in regions of Exon 1 (721) and Exon 2 (722) of the DNA base sequence may be encoded with the markers corresponding to the pair of the genotypes of the SNP data.

Referring to FIG. 5 again, the SNP data included in the reference data updated by the step 510 according to the exemplary embodiment may include a DNA base sequence of the corresponding genotype, information of the SNP included in the DNA base sequence of the gene, and markers inserted into the positions of the plurality of regions in the DNA base sequence. The markers inserted into the positions of the plurality of regions in the DNA base sequence may correspond to information of markers which encode the plurality of regions in the DNA base sequence.

According to the exemplary embodiment, the pair of the DNA base sequences of the SNP data included in the reference data 502 may be separated to be used. In other words, in the step 510 for updating the reference data 502 according to the exemplary embodiment, the insertion of the marker 503 corresponding to the pair of the genotypes of the SNP data into the plurality of predetermined regions included in the SNP data refers to the insertion of the marker 503 corresponding to the genotype of the DNA base sequence into a predetermined position in the plurality of predetermined regions, with respect to each of the DNA base sequences included in the SNP data. For example, a marker indicating a genotype of the corresponding DNA base sequence may be inserted in the middle of each exon region present in one DNA base sequence included in the reference data. The marker indicating the genotype of the DNA base sequence according to the exemplary embodiment may correspond to a form in which the binary markers corresponding to the plurality of genotypes form a tuple.

For example, the first SNP data may include an A type of first DNA base sequence and a B type of second DNA base sequence. In this case, a binary marker indicating the A type is inserted in a predetermined position (for example, a center position of exons) in exons included in the first DNA base sequence and a binary marker indicating the B type may be inserted in a predetermined position (for example, a center position of exons) in exons included in the second DNA base sequence. Here, the binary marker which indicates the specific genotype may include a tuple configured by binary markers corresponding to the genotypes of the gene on the subject of analysis. For example, when there are an A type, a B type, and a C type as genotypes of the gene on the subject of analysis, the binary marker indicating the A type corresponds to (1, 0, 0), the binary marker indicating the B type corresponds to (0, 1, 0), and the binary marker indicating the C type may correspond to (0, 0, 1).

According to the exemplary embodiment, the step 510 for updating the reference data 502 may include a step for inserting a maker 503 corresponding to the pair of the genotypes of the SNP data into one region of the plurality of predetermined regions included in the SNP data. For example, when the plurality of predetermined regions of the SNP data included in the reference data 502 is Exon 1 and Exon 2, the reference data updated in the step 510 may include SNP data in which the marker 503 is inserted only into the Exon 1 region and SNP data in which the marker 503 is inserted only into the Exon 2 region.

The step 520 for acquiring a second probability of corresponding to each genotype according to the exemplary embodiment may correspond to a step for acquiring second probabilities that the SNP data on the subject of analysis corresponds to each of the plurality of genotypes for the gene on the subject of analysis. In other words, the second probability that the SNP data on the subject of analysis corresponds to a specific genotype is a probability of corresponding to the corresponding genotype acquired based on the SNP data on the subject of analysis and the updated reference data and the step 520 may correspond to a step for acquiring a second probability for each of the plurality of genotypes for the gene on the subject of analysis.

According to the exemplary embodiment, the step 520 for acquiring a second probability of corresponding to each genotype may include a step for acquiring second probabilities that the SNP data on the subject of analysis corresponds to each genotype by inputting the SNP data on the subject of analysis and the reference data to an estimation model 505. The estimation model 505 according to the exemplary embodiment may correspond to a hidden Markov based model which receives the SNP data 501 on the subject of analysis and the reference data to output a result of calculating probabilities that the SNP data 501 on the subject of analysis corresponds to the plurality of genotypes predefined to the gene on the subject of analysis, for every region. To be more specific, the step 520 according to the exemplary embodiment may include a step for calculating probabilities that the SNP data on the subject of analysis corresponds to genotypes of the plurality of SNP data included in the reference data for every region by inputting the SNP data 501 on the subject of analysis and the updated reference data to the estimation model 505 and a step for acquiring second probabilities that the SNP data on the subject of analysis corresponds to each of the plurality of genotypes, on the basis of probabilities for every region.

According to the exemplary embodiment, in order to acquire the second probability, a genetic distance 504 may be given to the estimation model as an input value. The genetic distance according to the exemplary embodiment may include a genetic distance between markers which are defined to correspond to each of the plurality of predefined genotypes of the gene on the subject of analysis. In this case, the genetic distance between the markers includes a genetic distance between the DNA base sequences determined as a genotype corresponding to each marker.

The method for measuring a genetic distance according to the exemplary embodiment may include a step for sampling SNP data on the subject of analysis and a plurality of SNP data, a step for calculating an interstate transition probability corresponding to the plurality of genotypes in the hidden Markov model, based on the sampled data, and a step for acquiring a genetic distance between the states, by converting an interstate transition probability.

An algorithm which measures a transition probability according to the exemplary embodiment may include Baum-Welch algorithm. The step for acquiring an interstate genetic distance by converting the interstate transition probability according to the exemplary embodiment may correspond to a step for converting the interstate transition probability into an interstate genetic distance using the following Equation.

[Equation 1]
$$\tau = 1 - e^{-4Nr/H}$$

In Equation 1, $\tau$ is a transition probability between states calculated in the step for calculating a transition probability, r is a genetic distance, N is the number of effective population of a race corresponding to a subject of analysis (the number of effective population is known for every race. For example, the number of effective population of westerners may be set to 10,000), H corresponds to the number of states of the hidden Markov model. Each SNP data included in the sampled reference data according to the exemplary embodiment corresponds to SNP data extracted from one organism so that H may correspond to the number of organisms from which the SNP data included in the sampled reference data is extracted.

According to the exemplary embodiment, the second probability that the SNP data on the subject of analysis corresponds to each genotype is acquired in consideration of the genetic distance so that the accuracy of genotype prediction may be increased.

The estimation model 505 according to the exemplary embodiment may include BEAGLE model or an artificial neural network model. Hereinafter, the estimation model 505 will be described with the BEAGLE model as an example, but is not limited thereto.

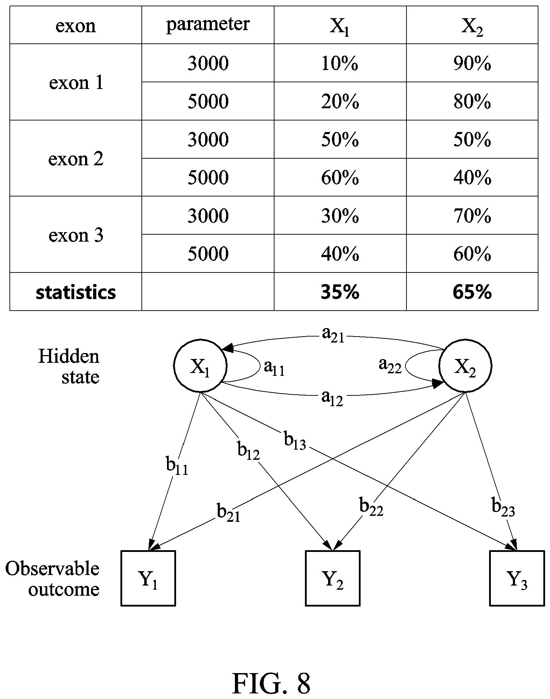

For example, referring to FIG. 8, the estimation model 505 according to the exemplary embodiment of FIG. 5 may include a model which predicts a genotype from the SNP data based on a hidden Markov model. In this case, the estimation model 505 may include hidden states X1, and X2 corresponding to a plurality of genotypes included in the gene on the subject of analysis, observable outcomes Y1, Y2, and Y3 corresponding to the SNP data, transition probabilities a11, a12, a21, and a22 between hidden states, and emission probabilities b11, b12, b13, b21, b22, and b23 between the hidden states and observable outcomes.

According to the exemplary embodiment, the reference data may be updated by inserting markers into a plurality of exon regions Exon 1, Exon 2, and Exon 3 included in the corresponding SNP data, so as to correspond to the SNP data included in the reference data. In this case, the estimation model according to the exemplary embodiment may calculate a probability of corresponding to genotypes X1 and X2 for each of the plurality of regions Exon 1, Exon 2, and Exon 3. In the step 520 of FIG. 5 according to the exemplary embodiment, the probability of corresponding to each genotype may be represented by an average of probabilities calculated for each of the plurality of regions.

According to the exemplary embodiment, in order to acquire the second probabilities, a step for setting a plurality of parameters indicating lengths of base sequences to analyze SNP data on the subject of analysis based on the plurality of SNP data included in the updated reference data, a step for calculating probabilities that the SNP data on the subject of analysis corresponds to genotypes of the plurality of SNP data for every combination of regions and the parameters, by inputting the SNP data on the subject of analysis, the updated reference data, and the parameters to the prediction model, and a step for acquiring a second probability of corresponding to each genotype of the SNP data on the subject of analysis, based on the probability for every combination may be included.

For example, referring to FIG. 8, the plurality of parameters indicating the lengths of the base sequences to analyze the SNP data on the subject of analysis may be set to 3000 and 5000. In this case, the estimation model according to the exemplary embodiment may calculate a probability of corresponding to genotypes X1 and X2, for each combination of the plurality of regions Exon 1, Exon 2, and Exon 3 and the parameters 3000 and 5000. For example, referring to FIG. 8, the probability of corresponding to the genotype X1 for each combination of the plurality of regions Exon 1, Exon 2, and Exon 3 and the parameters 3000 and 5000 may include a probability of 10% calculated by setting the Exon 1 region and the parameter of 3000 and a probability of 20% calculated by setting the Exon 1 region and the parameter of 5000.

Referring to FIG. 8, the probability of corresponding to X1 may be represented by 35% which is an average of the probabilities calculated for each combination of the plurality of regions Exon1, Exon 2, and Exon 3 and parameters 3000 and 5000 and a probability of corresponding to X2 may be represented by 65% which is an average of the probabilities calculated for each combination of the plurality of regions Exon1, Exon 2, and Exon 3 and parameters 3000 and 5000.

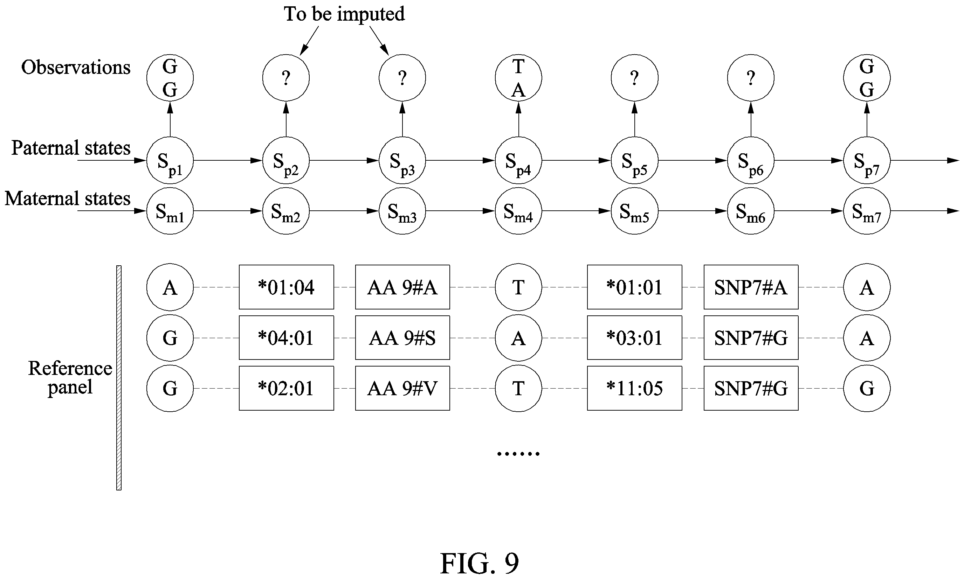

For the convenience of description, HMM having a general circular structure has been described in FIG. 8 as an example, but the HMM according to the exemplary embodiment may have a structure of FIG. 9. Referring to FIG. 9, the state may be transited from the left to the right by the genomic position.

The genotype predicting method according to the exemplary embodiment uses an NGS based method which predicts a genotype by mapping base sequences of the gene region of the NGS data on the subject of analysis to a reference genome whose genotype is determined and an SNP based method which predicts a genotype by an estimation model on the basis of reference data by extracting the SNP data in the intergenic region from the NGS data on the subject of analysis. Therefore, the NGS data on the subject of analysis according to the exemplary embodiment may include NGS data according to whole genome sequencing which sequences all the base sequences in the gene region and the intergenic region. Further, in the case of the NGS data according to exome sequencing or targeted sequencing, other than the whole genome sequencing, SNP near the gene region is counted and the SNP data extracted from the gene region is also used for the SNP based genotype prediction technique according to the exemplary embodiment. Therefore, the NGS data according to the exemplary embodiment is not necessarily limited to the NGS data according to the whole genome sequencing, but may include NGS data according to the exome sequencing or targeted sequencing.

The method according to the exemplary embodiment may be implemented as a program command which may be executed by various computers to be recorded in a computer readable medium. The computer readable medium may include solely a program command, a data file, and a data structure or a combination thereof. The program instruction recorded in the medium may be specifically designed or constructed for the exemplary embodiment or known to those skilled in the art of a computer software to be used. Examples of the computer readable recording medium include magnetic media such as a hard disk, a floppy disk, or a magnetic tape, optical media such as a CD-ROM or a DVD, magneto-optical media such as a floptical disk, and a hardware device which is specifically configured to store and execute the program command such as a ROM, a RAM, and a flash memory. Examples of the program command include not only a machine language code which is created by a compiler but also a high level language code which may be executed by a computer using an interpreter. The hardware device may operate as one or more software modules in order to perform the operation of the exemplary embodiment and vice versa.

The software may include a computer program, a code, an instruction, or a combination of one or more of them and configure the processing device to be operated as desired or independently or collectively command the processing device. The software and/or data may be permanently or temporarily embodied in an arbitrary type of machine, component, physical device, virtual equipment, computer storage medium, or device, or signal wave to be transmitted to be interpreted by a processing device or provide command or data to the processing device. The software may be distributed on a computer system connected through a network to be stored or executed in a distributed manner. The software and data may be stored in one or more computer readable recording media.

As described above, although exemplary embodiments have been described by limited drawings, those skilled in the art may apply various technical modifications and changes based on the above description. For example, even when the above-described techniques are performed by different order from the described method and/or components such as systems, structures, devices, or circuits described above are coupled or combined in a different manner from the described method or replaced or substituted with other components or equivalents, the appropriate results may be achieved.

Therefore, other implements, other embodiments, and equivalents to the claims are within the scope of the following claims.

**Claims**

1. A method for predicting genotype using NGS data, comprising:

   a step for acquiring next generation sequencing (NGS) data on a subject of analysis;
   a step for mapping the NGS data on the subject of analysis to base sequences having different genotypes for genes on the subject of analysis;
   a step for acquiring first probabilities that the NGS data on the subject of analysis correspond to genotypes for the genes on the subject of analysis on the basis of the mapping result;
   a step for extracting SNP data on the subject of analysis from the NGS data;
   a step for acquiring reference data including a plurality of SNP data having different genotypes for the genes on the subject of analysis;
   a step for acquiring second probabilities that the SNP data on the subject of analysis corresponds to each of the plurality of genotypes on the basis of the SNP data on the subject of analysis and the reference data; and
a step for predicting a genotype of the NGS data on the subject of analysis on the basis of the first probabilities and the second probabilities.

2. The method for predicting a genotype according to claim 1, wherein the step for acquiring first probabilities includes:

   a step for acquiring a length of a mapped base sequence in the NGS data with respect to each of base sequences having different genotypes for the gene on the subject of analysis; and
   a step for acquiring first probabilities of corresponding to each genotype for the gene on the subject of analysis, on the basis of the length of the mapped base sequence.

3. The method for predicting a genotype according to claim 1, wherein the step for predicting a genotype of the NGS data on the subject of analysis includes:

   a step for acquiring final probabilities that the SNP data on the subject of analysis corresponds each of the plurality of genotypes, by calculating a first probability and a second probability for every genotype; and
   a step for predicting a genotype corresponding to the highest final probability, among the final probabilities, as a genotype of the NGS data on the subject of analysis.

4. The method for predicting a genotype according to claim 1, wherein the step for extracting SNP data on the subject of analysis further includes:
   a step for detecting SNP from an intergenic region in the NGS data.

5. The method for predicting a genotype according to claim 1, wherein the step for acquiring reference data further includes:
   a step for inserting a marker corresponding to a genotype of the SNP data into each of a plurality of predetermined regions included in SNP data, with respect to each of the plurality of SNP data whose genotype for the gene on the subject of analysis is determined.

6. The method for predicting a genotype according to claim 1, wherein the step for acquiring reference data further includes:
   a step for inserting a binary marker corresponding to a genotype of the SNP data into each of a plurality of exons included in SNP data, with respect to each of the plurality of SNP data whose genotype for the gene on the subject of analysis is determined.

7. The method for predicting a genotype according to claim 1, wherein the step for acquiring second probabilities includes:

> a step for calculating probabilities that the SNP data on the subject of analysis corresponds to the genotypes of the plurality of SNP data, for every region, by inputting the SNP data on the subject of analysis and the reference data to an estimation model; and
> a step for acquiring second probabilities that the SNP data on the subject of analysis corresponds to each of the plurality of genotypes, on the basis of probabilities for every region.

8. The method for predicting a genotype according to claim 1, wherein the step for acquiring second probabilities includes:

> a step for calculating a genetic distance between a plurality of markers corresponding to the plurality of genotypes; and
> a step for acquiring second probabilities that the SNP data on the subject of analysis corresponds to each of the plurality of genotypes on the basis of the SNP data on the subject of analysis, the reference data, and the genetic distance.

9. The method for predicting a genotype according to claim 1, wherein the step for acquiring second probabilities includes:

> a step for sampling the SNP data on the subject of analysis and the plurality of SNP data;
> a step for calculating an interstate transition probability corresponding to the plurality of genotypes in the hidden Markov model, on the basis of the sampled data;
> a step for acquiring an interstate genetic distance by converting the interstate transition probability; and
> a step for acquiring second probabilities that the SNP data on the subject of analysis corresponds to each of the plurality of genotypes on the basis of the genetic distance, the reference data, and the SNP data on the subject of analysis.

10. The method for predicting a genotype according to claim 1, wherein the SNP data on the subject of analysis includes:

> at least some of DNA base sequences of a user on a subject of analysis; and
> information of the at least some SNP included in the at least some DNA base sequences.

11. The method for predicting a genotype according to claim 1, wherein each SNP data included in the reference data includes:

> a DNA base sequence of a corresponding genotype;
> information of SNP included in the DNA base sequence; and
> markers inserted into a plurality of predetermined regions in the DNA base sequence.

12. The method for predicting a genotype according to claim 1, wherein the gene on the subject of analysis is a HLA gene,

> the plurality of genotypes includes a plurality of genotypes defined in the HLA gene, and
> the NGS data on the subject of analysis includes a base sequence of the HLA gene.

13. A computer program stored in a medium to be coupled to hardware to execute the method according to any one of claims 1 to 12.

14. A device for predicting a genotype using NGS data, comprising:

> a memory which stores reference data including a plurality of SNP data in which a genotype of a gene on a subject of analysis is determined; and
> at least one processor which acquires next generation sequencing (NGS) data on the subject of analysis, maps the NGS data on the subject of analysis to base sequences having different genotypes for genes on the subject of analysis, acquires first probabilities that the NGS data on the subject of analysis correspond to genotypes for the genes on the subject of analysis on the basis of the mapping result, extracts SNP data on the subject of analysis from the NGS data, acquires reference data including a plurality of SNP data into which a marker corresponding to a genotype for the gene on the subject of analysis is inserted, acquires second probabilities that the SNP data on the subject of analysis corresponds to each of the plurality of genotypes on the basis of the SNP data on the subject of analysis and the reference data, and predicts a genotype of the NGS data on the subject of analysis on the basis of the first probabilities and the second probabilities.

15. The device for predicting a genotype according to claim 14, wherein when the first probabilities are acquired, the processor acquires a length of the mapped base sequence in the NGS data, with respect to each of base sequences having different genotypes for the gene on the subject of analysis and acquires the first probabilities of corresponding to each genotype for the gene on the subject of analysis, on the basis of the length of the mapped base sequence.

**16.** The device for predicting a genotype according to claim 14, wherein when the genotype of the NGS data on the subject of analysis is predicted, the processor acquires final probabilities that the SNP data on the subject of analysis corresponds each of the plurality of genotypes, by calculating a first probability and a second probability for every genotype and predicts a genotype corresponding to the highest final probability, among the final probabilities, as a genotype of the NGS data on the subject of analysis.

**17.** The device for predicting a genotype according to claim 14, wherein when the SNP data on the subject of analysis is extracted, the processor detects SNP from an intergenic region in the NGS data.

**18.** The device for predicting a genotype according to claim 14, wherein when the reference data is acquired, the processor inserts a marker corresponding to a genotype of the SNP data into each of a plurality of predetermined regions included in SNP data, with respect to each of the plurality of SNP data whose genotype for the gene on the subject of analysis is determined.

**19.** The device for predicting a genotype according to claim 14, wherein when the second probabilities are acquired, the processor calculates probabilities that the SNP data on the subject of analysis corresponds to the genotypes of the plurality of SNP data, for every region, by inputting the SNP data on the subject of analysis and the reference data to an estimation model and acquires second probabilities that the SNP data on the subject of analysis corresponds to each of the plurality of genotypes, on the basis of probabilities for every region.

**20.** The device for predicting a genotype according to claim 14, wherein when the second probabilities are acquired, the processor calculates a genetic distance between a plurality of markers corresponding to the plurality of genotypes and acquires second probabilities that the SNP data on the subject of analysis corresponds to each of the plurality of genotypes on the basis of the SNP data on the subject of analysis, the reference data, and the genetic distance.

**21.** The device for predicting a genotype according to claim 14, wherein the gene on the subject of analysis is a HLA gene, the plurality of genotypes includes a plurality of genotypes defined in the HLA gene, and the NGS data on the subject of analysis includes a base sequence of the HLA gene.

FIG. 1

FIG. 2

EP 3 975 189 A1

FIG. 3

FIG. 4

FIG. 5

FIG. 6

710

700
Chromosome

Intergenic Region

720

Gene

Gene

722

721

5'UTR

Exon

Intron

Exon

3'UTR

FIG. 7

| exon | parameter | $X_1$ | $X_2$ |
|------|-----------|-------|-------|
| exon 1 | 3000 | 10% | 90% |
|        | 5000 | 20% | 80% |
| exon 2 | 3000 | 50% | 50% |
|        | 5000 | 60% | 40% |
| exon 3 | 3000 | 30% | 70% |
|        | 5000 | 40% | 60% |
| **statistics** | | **35%** | **65%** |

FIG. 8

FIG. 9

## INTERNATIONAL SEARCH REPORT

| | |
|---|---|
| | International application No. |
| | **PCT/KR2020/006720** |

**A. CLASSIFICATION OF SUBJECT MATTER**

*G16B 25/10(2019.01)i, G16B 20/20(2019.01)i, G16B 30/10(2019.01)i*

According to International Patent Classification (IPC) or to both national classification and IPC

**B. FIELDS SEARCHED**

Minimum documentation searched (classification system followed by classification symbols)
G16B 25/10; G06F 19/20; G06F 19/22; G06F 19/24; G16B 20/20; G16B 30/10

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: NGS, data, genotype, HLA

**C. DOCUMENTS CONSIDERED TO BE RELEVANT**

| Category* | Citation of document, with indication, where appropriate, of the relevant passages | Relevant to claim No. |
|---|---|---|
| A | 국승호. 정확한 HLA 유전형 예측을 위한 소프트웨어 도구. 석사학위논문, 울산대학교대학원. December 2018, pages 1-40 (KOOK, Seungho. Software Tools for Accurate HLA Prediction. Master's thesis, Graduate School of Ulsan University.) See chapter 2. | 1-21 |
| A | COOK, S. et al. CookHLA: Accurate HLA Imputation from Genomic Data. The 27th International KOGO Annual Conference. 05 September 2018, Sejong Convention Center, Sejong University, Seoul, Korea. poster I-11 See abstract. | 1-21 |
| A | COOK, S. et al. CookHLA(v0.3): Accurate, Efficient, and Memory-Efficient HLA Imputation. The 14th KOGO Winter Symposium. 05 February 2018, 대명리조트 비발디파크, 홍천, 강원도, 대한민국 (Daemyung Resort Vivaldi Park, Hongcheon, Gangwon-do, Korea). poster I-05 See abstract. | 1-21 |
| A | COOK, S. et al. PgmNr 1557: CookHLA: Accurate HLA imputation. ASHG 2018 Annual Meeting. 24 January 2019, the posters See abstract. | 1-21 |
| A | KR 10-2016-0082715 A (INDUSTRY-ACADEMIC COOPERATION FOUNDATION, YONSEI UNIVERSITY) 11 July 2016 See the entire document. | 1-21 |

☒ Further documents are listed in the continuation of Box C.     ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 28 AUGUST 2020 (28.08.2020) | **28 AUGUST 2020 (28.08.2020)** |

| Name and mailing address of the ISA/KR | Authorized officer |
|---|---|
| Korean Intellectual Property Office Government Complex Daejeon Building 4, 189, Cheongsa-ro, Seo-gu, Daejeon, 35208, Republic of Korea Facsimile No. +82-42-481-8578 | Telephone No. |

Form PCT/ISA/210 (second sheet) (January 2015)

**INTERNATIONAL SEARCH REPORT**

| International application No. |
|---|
| **PCT/KR2020/006720** |

| C (Continuation). | DOCUMENTS CONSIDERED TO BE RELEVANT | |
|---|---|---|
| Category* | Citation of document, with indication, where appropriate, of the relevant passages | Relevant to claim No. |
| A | KR 10-2018-0090680 A (ELECTRONICS AND TELECOMMUNICATIONS RESEARCH INSTITUTE) 13 August 2018<br>See the entire document. | 1-21 |

Form PCT/ISA/210 (continuation of second sheet) (January 2015)

INTERNATIONAL SEARCH REPORT
Information on patent family members

International application No.

**PCT/KR2020/006720**

| Patent document cited in search report | Publication date | Patent family member | Publication date |
|---|---|---|---|
| KR 10-2016-0082715 A | 11/07/2016 | KR 10-1638473 B1 | 12/07/2016 |
| KR 10-2018-0090680 A | 13/08/2018 | None | |

Form PCT/ISA/210 (patent family annex) (January 2015)